# EUROPEAN PATENT APPLICATION

(11) **EP 1 356 846 A1**
(43) Date of publication of application: **29.10.2003**
(21) Application number: 03450070.2
(22) Date of filing: 18.03.2003
(51) Int. Cl.: A61M 35/00, A61M 37/00, A61F 5/41

(54) **Device for delivering liquid medications, nutrients or gases to local tissue**

(30) Priority: 24.04.2002 US 131062
(71) Applicant: Johnson, Lanny L., Okemos, Michigan 48864 (US)
(72) Inventor: Johnson, Lanny L., Okemos, Michigan 48864 (US)
(74) Representative: Haffner, Thomas M.

(57) **Abstract**

A delivery member having a flexible wall is formed to substantially surround vessel in contacting relationship wherewith. The delivery member contains pressurized medication, nutrients or gas. A plurality of small openings are provided in that portion of the wall which contacts the tissue to permit the medication, nutrients or gas to escape from the member and perfuse into the tissue.

## Description

### BACKGROUND OF THE INVENTION

### 1. Field of the Invention

The present invention relates to an arrangement for delivering liquid medications, nutrients or gases to local tissue by pressure perfusion.

### 2. Prior Art

Liquid medications or nutrients typically are delivered by oral ingestion, subcutaneous or intramuscular injection, or intravenously. As alternatives to such delivery devices, various "needleless" hypodermic injection devices have been developed. Such devices are characterized by the delivery of medications or nutrients under high pressure in a very localized area.

Applicant's co-pending application, Serial No. 10/043,505, filed on January 9, 2002, discloses non-invasive delivery devices particularly suitable for directing gas or liquid medications or nutrients to tissue. More particularly, a fluted delivery member is secured to a conventional syringe or gas supply container to receive liquid or gas discharged therefrom. The interior of the fluted member includes an open-ended bell-shaped chamber portion. With the open end of the fluted member pressed against or is secured to the area to which the liquid or gas is to be delivered, and with the liquid or gas being discharged into the chamber until it becomes full and pressurized, the liquid or gas is able to enter the tissue by perfusion and to disuse throughout neighboring tissue.

### SUMMARY OF THE INVENTION

While the invention disclosed in application Serial No. 10/043,505 may be used to deliver liquid medications or nutrients, or gases, to tubular tissue(such as nerve, artery, vein, bowel and the like), an alternative to such a delivery arrangement is disclosed in the present application. More particularly, a flexible member substantially surrounds the tubular tissue. The member is provided with small openings positioned in facing relationship with the tissue. When the member is pressurized as a result of a sufficient amount of medication, nutrient or gas being introduced within the member, the openings contact the tissue, and the liquid or gas enters the tissue by perfusion and diffuses throughout the neighboring tissue.

### BRIEF DESCRIPTION OF THE DRAWINGS

The invention now will be described in further detail with respect to the accompanying drawings, wherein:
FIG.1 is a perspective view of a first embodiment of the invention;
FIG. 2 is a sectional view taken along line 2-2 of FIG. 1;
FIG. 3 is a perspective view of a second embodiment of the invention; and
FIG. 4 is a sectional view taken along line 4-4 of FIG. 4.

### DETAILED DESCRIPTION OF PREFERRED EMBODIMENTS OF THE INVENTION

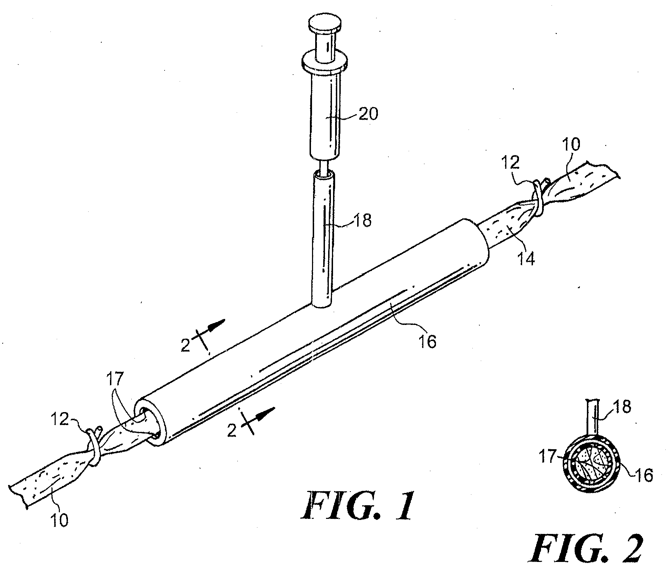

Referring now to the drawings, FIG. 1 illustrates a first embodiment of the invention. A length of tubular tissue 10 is shown in the form of an artery, vein or other fluid-carrying organ to which temporary constriction devices 12 are attached so as to define a target area 14 for the tissue to which liquid medication, nutrients or a gas is to be directed. A member 16 is formed cylindrically to substantially surround the tissue 10 in the target area. Member 16 comprises a flexible wall provided with small openings 17 on the wall surface facing the target area 14 of the tissue (FIG. 2). The member 16 on its outer surface is provided with an inlet tube 18 joined to a container 20 carrying the liquid medication, nutrients or gas. In the illustration, container 20 is shown as a conventional syringe typically used for dispensing liquid medications or nutrients.

Container 20 discharges its contents through inlet tube 18 so as to introduce liquid medication, nutrients or gas into member 16. Because the openings 17 in member 16 are small, the material introduced into member 16 becomes increasingly pressurized until the material is able to pass through the openings and enter the tissue by perfusion and then diffuse throughout the target area.

When the desired amount of medication, nutrient or gas has been supplied to the tissue, member 16 and the constriction devices are removed.

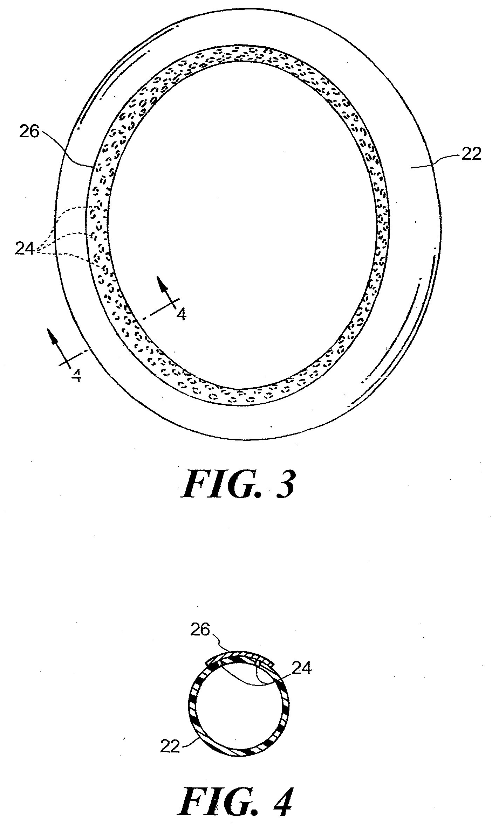

The concept employed in the arrangement just described can be used in maintaining penis erection. This is illustrated in FIGs. 3 and 4 wherein a toroid 22 formed from flexible material and containing pressurized gas (for example, nitric oxide) is positioned about the proximal end of the penis. The toroid contains a number of small openings 24 (FIG. 4) positioned along the interior circumference of the toroid. Openings 24 are covered by a removable tape 26 in order to retain the pressurized gas within the toroid. When the toroid is positioned on the penis as illustrated and the tape is removed, the openings 24 contact the user's skin.

The toroid 22 serves a first purpose of constricting blood outflow from an erected penis. Additionally, when toroid 22 is compressed, either by hand or as a result of natural compression occurring during the coitus act, the nitric oxide gas passes through the openings 24 and perfuses through the skin at the base of the penis and then diffuses within the penis. The introduction of nitric oxide to the penis enhances maintenance of erection.

The device illustrated in FIGs. 3 and 4 can be used with or without a condum.

## Claims

1. A device for delivering liquid medication, nutrients or gas to tubular body tissue, comprising:
a delivery member formed to substantially surround said tubular tissue, said member having: a flexible wall to permit inflation and pressurized retention of medication, nutrients or gas introduced within the member; and a plurality of openings in said wall positioned to contact the tissue when the member is pressurized.

2. A device according to claim 1, wherein said member, when substantially surrounding the tissue, has a cylindrical shape.

3. A device according to claim 1, wherein said member, when substantially surrounding the tissue, has a toroidal shape.

4. A device according to claim 2, further comprising an inlet formed in said wall through which medication, nutrients or gas is introduced into said member.

5. A device according to claim 4, wherein said inlet is adapted to cooperate with a syringe.

6. A device according to claim 3, further comprising a removable tape disposed on said wall and covering the openings to prevent pressurized medication, nutrients or gas from escaping from the delivery member until said tape is removed from the wall.
